# EUROPEAN PATENT SPECIFICATION

(11) **EP 1 933 148 B1**
(45) Date of publication and mention of the grant of the patent: **25.08.2010**
(21) Application number: 06025531.2
(22) Date of filing: 11.12.2006
(51) Int. Cl.: G01N 33/558, G01N 33/543, G01N 33/58, C12Q 1/68

(54) **Urinary immunochromatographic multiparameter detection cup**
Gefäss zur immunochromatographischen Detektion mehrerer Parameter in Urin
Récipient pour la détection immunochromatographique de plusieurs paramètres urinaires

(43) Date of publication of application: 18.06.2008
(73) Proprietor: AraGen Biotechnology Co. Ltd., 11710 Naor (JO)
(72) Inventor: Badwan, Adnan, Dr., 11185 Amman (JO); Murshed, Abedel-qader Mohammed, 11118 Amman (JO)
(74) Representative: Winkler, Andreas Fritz Ernst

(56) References cited:
- EP-A1- 0 698 792
- EP-A1- 0 905 517
- EP-A1- 1 215 496
- WO-A-01/61347
- WO-A-02/42768
- WO-A-95/07659
- US-B1- 6 616 893
- OKU Y ET AL: "Development of oligonucleotide lateral-flow immunoassay for multi-parameter detection" JOURNAL OF IMMUNOLOGICAL METHODS, vol. 258, no. 1-2, 1 December 2001 (2001-12-01), pages 73-84, XP004311918
- SUGUNAN ET AL: "Heavy-metal ion sensors using chitosan-capped gold nanoparticles" SCIENCE AND TECHNOLOGY OF ADVANCED MATERIALS, vol. 6, no. 3-4, April 2005 (2005-04), pages 335-340, XP005039763

## Description

The present invention refers to an immuno-chromatographic detection cup and its use for simultaneously detecting at least one antigen, e.g. tuberculosis antigen, malaria antigen and pneumonia antigen, and at least one antibody, e.g. HIV antibody, HCV antibody and *H pylori* antibody, in a urine sample. The detection makes use of gold-labelled antibodies and antigens, oligo-nucleotide linked antibodies and antigens, and complementary oligo-nucleotides for capturing the linked antibodies.

### Background of the Invention

Testing for HIV is an essential component in the diagnosis and treatment of persons infected with the virus, in screening of blood for transfusion, in surveillance and in HIV/AIDS related research. Thus, accurate and cost-effective testing is of great importance in combating the spread of HIV. It is imperative that tests for the diagnosis of HIV infection should be as accurate as possible, given the serious ethical, legal and social issues that accompany HIV infection ¹.

The use of body fluids other than blood as specimens for detecting antibodies to HIV has been reported to have potential as an alternative medium for HIV testing. The use of urine samples may be attractive due to the ease of sample collection, possible cost savings, better safety (against needle stick injuries) and higher compliance rates. Assays for these types of specimens can be a useful alternative when it is difficult or impossible to test for HIV in blood samples. It may be that blood can not be drawn for religious reasons or difficulties may be experienced in collecting blood samples in hard to reach places where it is, nevertheless, important to have epidemiological surveillance¹. Moreover, urinary samples are the more safety type of specimens in case of HIV infection.

Today, new HIV tests have been developed for use with urine specimens. Urine contains very low levels of IgG, frequently around 1 mg/l, and therefore very sensitive techniques are required to detect specific antibody. Thus, to date, the tests for urine specimens have been based on ELISA and Western blot techniques, however, efforts are being made to develop simple and/or rapid tests for the detection of antibody to HIV in urine specimens¹. It would be of particular advance to have rapid immuno-chromatographic test devices available for detecting HIV antibodies in urine.

In addition to HIV infection, tuberculosis (TB) is an important and increasing public health problem in both industrialized and developing countries. Hence, the development of new inexpensive, rapid and field adapted methods for its diagnosis is urgently needed. Sputum culture, which is still the reference method for the diagnosis of pulmonary TB, is cumbersome and time-consuming, and requires access to expensive biosafety level 3 (BSL3) laboratories. Microscopy of direct smears for acid-fast bacilli (AFB) as recommended by WHO for developing countries is the most commonly used method for diagnosis of TB. A major disadvantage with this method is its low sensitivity, even after concentration of the sputum samples.

The availability of new field adapted, low-cost, and rapid diagnostic tests to supplement AFB microscopy, and especially methods improving the diagnosis in AFB-negative disease, would be of great benefit for TB control programs, in particular in areas lacking appropriate safety laboratories. Among the newly developed methods for rapid diagnosis of TB, nucleic acid amplification methods such as PCR seem most promising, but the technology is still too complex to be feasible for TB control programs in developing countries. Antibodies against a number of mycobacterial antigens have been identified in patients using a variety of immunological techniques, but no antibody test has so far reached sufficient sensitivity and/or specificity for routine diagnostic purposes. Detection of circulating or secreted *Mycobacterium tuberculosis* antigens seems attractive and has been explored in a number of studies. However, no satisfactory commercial test for mycobacterial antigens in serum or sputum is currently available.

The idea of identifying mycobacterial antigens in urine of TB patients is attractive for several reasons: urine is more readily obtainable than serum samples and urinary specimens do not carry the risks inherent to needles and blood-based laboratory work. Furthermore, if the urine specimens are boiled before handling, there is no need for BSL3 facilities.

In 1920s, mycobacterial antigens were detected in the urine of TB patients, and the diagnostic potential of such antigens was subsequently discussed by other scientists. More recently, the diagnostic value of mycobacterial antigens in the urine of leprosy patients has been assessed.

Unfortunately, the techniques involved turned out to be insufficiently sensitive in paucibacillary patients, the patient group where improved diagnostic tests are needed most

Lipoarabinomannan (LAM) is a major and structurally important glycolipid component of the outer cell wall of all mycobacteria and may account for up to 15% of the total bacterial weight. LAM is a carbohydrate antigen with glycosidic linkages for which no human degrading glycosidases are known. Hence, we assumed that in active mycobacterial disease LAM may be cleared through the kidneys and occur in urine in antigenically intact form. Furthermore, since LAM is a carbohydrate antigen and thus inherently heat-stable, LAM may be detectable by sensitive immunological techniques even after boiling of the urine. At least theoretically, the amount of LAM in the urine should reflect the bacterial load, metabolic activity and/or rate of degradation of the bacteria, and hence permit a semi-quantitative assessment of the infectious status. A high sensitive, simple, fast and reliable method for LAM detection and quantification was reported using an enzyme-linked immunosorbent assay (ELISA) in AFB positive sputa from TB patients². However, it would be of particular advantage to have rapid immuno-chromatographic test devices available for detecting LAM in urine.

In recent years, the *in vitro* diagnostics (IVD) industry has made enormous efforts to develop immuno-chromatographic tests. Such tests have found applications in both clinical and non-clinical fields³. A clinical utility of this test format has been shown for more than 150 different analytes, and many of them are target now of commercially available diagnostic products ⁴. The wide range of applications for such devices has been reviewed^{3,5}.

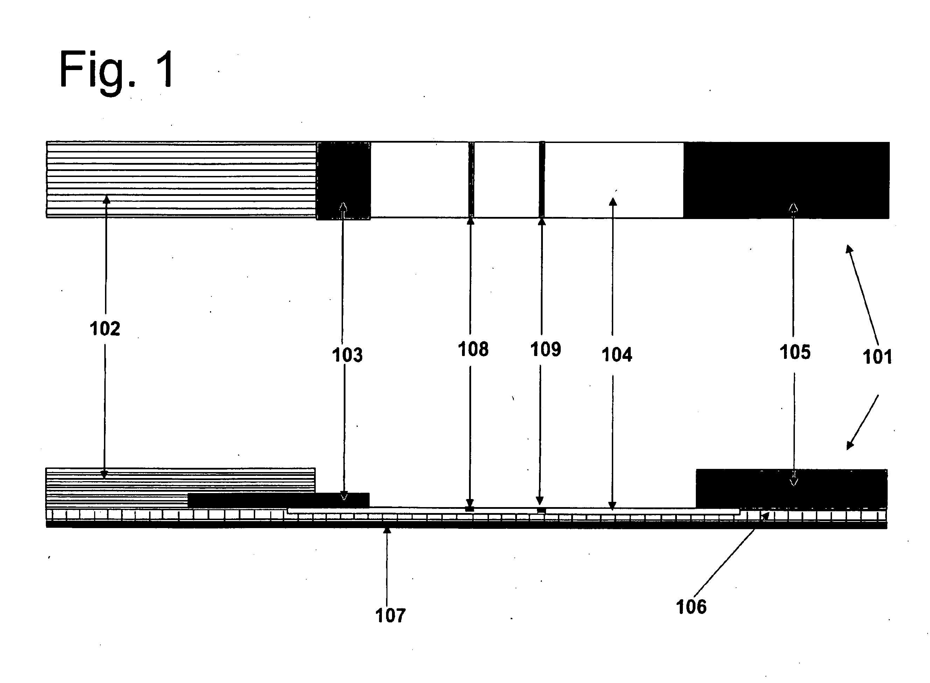

Rapid immuno-chromatographic test devices, e.g. in the form of a test strip, are made up of a number of components (see Fig. 1). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102. The sample then flows through the sample pad 102 into the conjugate pad 103, where it mixes with and releases the detector reagent. This mixture then flows across the membrane 104, where it binds with the test and control reagents located in the capture test zone 108 (sample zone) and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Using rapid immuno-chromatographic test devices, a physician can instantly not only examine a sample by himself, but also diagnose a patient on the basis of the obtained results. Such test devices for diagnostic purposes are easy to operate and thus do not only contribute to the comfort of professional users, e.g. medical stuff, but also allow the operation by non-professionals users, e.g. most patients.

However, a main demerit is that one immuno-chromatographic test device only detects one parameter at a time with the consequence that many different rapid immuno-chromatographic test devices are needed to check the suspected parameters, resulting in the requirement of enormous sample volume to be obtained from a patient as well as cumbersome test procedures.

WO 01/61347 discloses a urine collection cup device comprising a transparent cup for containing the fluid and a reagent strip for indicating the presence of a specific component of the fluid. The device further comprises a congregate pad disposed at the strip one end and end pads which are absorbent. The reagent strip comprises an absorbent membrane with a sample introduction portion and a test results portion. A conjugate pad containing a suitable conjugate is provided on the sample introduction portion. In addition, the test results portion may have incorporated therein a specific reagent which manifests a detectable response, for example a color change in the presence of a specific component of the fluid that is absorbed by the membrane.

US 6,616,893 discloses a diagnostic testing kit for collection of fluid samples comprising a reservoir cup insertable into a container vessel. Fluid placed in the cup is communicated to reactive test strips located in a separation cavity between the cup and the container vessel when assembled. The test strips have a reactive agent impregnated in them that reacts with specific substances in the fluid and provides a visual result through the sidewall of the container vessel to the user.

WO 95/07659 discloses an assaying device for collecting a sample comprising container means and assay means for chemically analyzing the sample. In one embodiment, the assaying means comprises a plurality of separated thin layer chromatograph strips, each strip comprising means for chemically analyzing the sample for a different specific substance thereby enabling up to five analyte specific tests to be run concurrently. The assaying means further comprises wick means for evenly distributing the sample portion to each of the plurality of separated thin layer chromatograph strips.

WO 02/42768 discloses a device for the analytical determination of a compound in a liquid such as urine comprising a cup and, in a vertical position, at least one dip strip.

Oku *et al*. (2001) ⁶ discloses a rapid oligonucleotide lateral-flow immunoassay, using a colloidal gold as an indicator, for the simultaneous detection of antigens and/or antibodies in specimen. This system can detect more than two types of antigens and/or antibodies in a single assay device at the same time. The device is basically composed of colloidal gold-labeled antibodies and oligonucleotide-labled antibodies fixed in a conjugate pad, and the complementary oligonucleotide-labeled proteins are immobilized on a nitrocellulose membrane. If the target antigen is present in a specimen, the colloidal gold-labeled antibody and oligonucleotide-labeled antibody will make a complex with the antigen. Subsequently, the formed complex migrates to the place where complementary oligonucleotide is immobilized and is bound to the solid phase via the DNA-DNA interaction. As a result, more than two types of reactions can be detected on a single assay device by the combination of colloidal gold-labeled antibodies, different oligonucleotide-labeled antibodies and complementary oligonucleotide-labeled proteins immobilized at different places on a nitrocellulose membrane.

EP 1 215 496 relates to kits and methods for detecting or measuring an analyte having bivalent or higher binding capability and contained in a liquid sample.

EP 0 905 517 discloses an assay method capable of simultaneously determining the presence or absence of one or more species of biological substances in a fluid sample or assaying the amounts thereof with a single assay device. The method involves contacting a liquid sample containing one or more species of analytes with a reagent including one or more species of marker-labeled ligands and one or more species of nucleic-acid labeled ligands to generate one or more species of complexes and capturing the complexes through complementary nucleic acids.

EP 0 698 792 relates to an assay reagent and kit for assaying more than one immunological ligands.

Sugunan *et al*. (2005) ⁸ discloses the use of gold nanoparticles capped with chitosan for sensing ions of heavy metals. The polycationic nature of chitosan enables attachment of the polymer to the negatively charged gold nanoparticle surfaces through electrostatic interactions. Use of chitosan serves dual purpose of ensuring stability of the colloid and also to functionalize the nanoparticles for use as sensors due to their optical properties.

It is an object of the present invention to provide a rapid immuno-chromatographic test device for detecting different analytes (antibodies and antigens) in urine in a single assay at the same time.

### Summary of the Invention

The object of the present invention is solved by a urinary immuno-chromatographic detection cup comprising:
(a) a sample-collecting container;
(b) at least one conjugate releasing pad, which conjugate releasing pad comprises at least one gold labelled antibody X and at least one oligo-nucleotide linked antibody X', wherein both antibodies are directed against the same antigen, and at least one gold labelled antigen Y and at least one oligo-nucleotide linked antigen Y', wherein both antigens are recognized by the same antibody;
(c) at least one test means inside the container separated from the conjugate releasing pad, which test means comprises at least one region comprising an oligo-nucleotide complementary to the oligo-nucleotide linked to the antibody X', at least one region comprising an oligo-nucleotide complementary to the oligo-nucleotide linked antigen Y', and at least one region comprising a control antibody and/or a control antigen;
(d) at least two sample absorbent pads linked to the test device at different positions, wherein a first sample absorbent pad is located at the bottom, and a second sample absorbent pad is located at the top of the container, and wherein the sample absorbent pad at the top has a capacity of absorbing at least 15 ml of sample.

Antibodies X and X' may be identical or different, as long as they are directed against the same antigen. Antigens Y and Y' may be identical or different as long as they are recognized by the same antibody;

In one embodiment, the antibodies X and X' are directed against an antigen selected from tuberculosis antigen, malaria antigen and pneumonia antigen.

In a preferred embodiment, the tuberculosis antigen is LAM, the malaria antigen is HRP-II, and the pneumonia antigen is PLY.

In one embodiment, the control antibody is anti-mouse IgG.

In one embodiment, the antigens Y and Y' are selected from envelop protein gp 160 from HIV, NS3 antigen from HCV, and *H. pylori* antigen.

In one embodiment, the test device further comprises a nitrocellulose membrane on which the complementary oligo-nucleotides are immobilized.

In one embodiment, the conjugate releasing pad is located at the internal surface of the container wall.

In a preferred embodiment, the sample absorbent pad at the top is fitted into a container cap.

In one embodiment, the conjugate releasing pad further comprises chitosan, which is optionally modified.

The object of the present invention is further solved by a use of the urinary immuno-chromatographic detection cup according to the present invention for detecting at least one antigen selected from tuberculosis antigen, malaria antigen and pneumonia antigen in a sample of urine of a subject, preferably human, and at least one antibody selected from HIV antibody, HCV antibody and *H*. *p*ylori antibody:

In one embodiment of the use, the volume of the urine sample is approximately 15 ml.

The object of the present invention is further solved by a method for preparing the urinary fluid immuno-chromatographic detection cup according to the present invention, wherein water-soluble chitosan, which is optionally modified is added during the preparation of gold labelled antibody X or gold labelled antigen Y prior to conjugation of colloid gold with the antibody or antigen.

In one embodiment of the method, the colloidal gold is prepared by reduction of 1% aqueous solution of tetra-chloroauric acid using tri-sodium citrate aqueous solution to produce spheroidal gold particles.

The object of the present invention is also solved by a urinary immuno-chromatographic detection cup comprising:
(a) a sample-collecting container;
(b) at least one conjugate releasing pad, which conjugate releasing pad comprises at least two different gold labelled antibodies (consider as anti-A, anti-B) and at least two different oligo-nucleotide (consider as 1 and 2) linked antibodies (consider as anti-A~1 and anti-B~2);
(c) at least one test strip inside the container separated from the conjugate releasing pad, the nitrocellulose membrane of this test strip printed with at least two lines of different oligo-nucleotides (consider as 1' and 2') complementary to each of the oligo-nucleotides linked to the antibodies, and at least a third line of one control antibody (non-specific binding of anti-A and anti-B);
(d) at least two sample absorbent pads linked to the test device at different positions, wherein a first sample absorbent pad is located at the bottom, and a second sample absorbent pad is located at the top of the container, and wherein the sample absorbent pad at the top has a capacity of absorbing at least 15 ml of sample.

The object of the present invention is also solved by a urinary immuno-chromatographic detection cup comprising:
(a) a sample-collecting container;
(b) at least one conjugate releasing pad, which conjugate releasing pad comprises at least two different gold labelled antigens (consider as C, D) and at least two different oligo-nucleotide (consider as 3 and 4) linked antigens (consider as C~3 and D~4);
(c) at least one test strip inside the container separated from the conjugate releasing pad, the nitrocellulose membrane of this test strip printed with at least two lines of different oligo-nucleotides (consider as 3' and 4') complementary to each of the oligo-nucleotides linked to the antigens, and at least a third line of one control antigen (non-specific binding of C and D);
(d) at least two sample absorbent pads linked to the test device at different positions, wherein a first sample absorbent pad is located at the bottom, and a second sample absorbent pad is located at the top of the container, and wherein the sample absorbent pad at the top has a capacity of absorbing at least 15 ml of sample.

The object of the present invention is also solved by a urinary immuno-chromatographic detection cup comprising:
(a) a sample-collecting container;
(b) at least one conjugate releasing pad, which conjugate releasing pad comprises at least one gold labelled antibody (consider as anti-E), at least one oligo-nucleotide (consider as 5) linked antibody (consider as anti-E~5), at least one gold labelled antigen (consider as F) and at least one oligo-nucleotide (consider as 6) linked antigen (consider as F~6);
(c) at least one test strip inside the container separated from the conjugate releasing pad, the nitrocellulose membrane of this test strip printed with at least one line of oligo-nucleotide (consider as 5') complementary to the oligo-nucleotide linked to the antibody, at least one oligo-nucleotide (consider as 6') complementary to the oligo-nucleotide linked antigen, at least a third line of one control antibody and antigen (non-specific binding of F and anti-E);
(d) at least two sample absorbent pads linked to the test device at different positions, wherein a first sample absorbent pad is located at the bottom, and a second sample absorbent pad is located at the top of the container, and wherein the sample absorbent pad at the top has a capacity of absorbing at least 15 ml of sample.

In one embodiment, the two antibodies are directed against two different antigens selected from tuberculosis antigen, malaria antigen and pneumonia antigen.

In a preferred embodiment, the tuberculosis antigen is LAM, the malaria antigen is HRP-II, and the pneumonia antigen is PLY.

In one embodiment, the control antibody is anti-mouse IgG.

In one embodiment, the two different antigens are selected from envelop protein gp 160 from HIV, NS3 antigen from HCV, envelop antigen from HCV, and *H. pylori* antigen,

In one embodiment, the test device further comprises a nitrocellulose membrane on which the complementary oligo-nucleotides are immobilized.

In one embodiment, the conjugate releasing pad is located at the internal surface of the container wall.

In a preferred embodiment, the sample absorbent pad at the top is fitted into a container cap.

In one embodiment, the conjugate releasing pad further comprises chitosan, which is optionally modified.

The object of the present invention is further solved by a use of the urinary immuno-chromatographic detection cup according to the present invention for detecting at least two different antigens selected from tuberculosis antigen, malaria antigen and pneumonia antigen in a sample of urine of a subject, preferably human.

In a preferred embodiment, the tuberculosis antigen is LAM, the malaria antigen is HRP-II, and the pneumonia antigen is PLY.

The object of the present invention is further solved by a use of the urinary immuno-chromatographic detection cup according to the present invention for detecting at least two different antibodies selected from HIV, HCV and *H. pylori* antibody in a sample of urine of a subject, preferably human.

The object of the present invention is further solved by a use of the urinary immuno-chromatographic detection cup according to the present invention for detecting at least one antigen selected from tuberculosis antigen, malaria antigen and pneumonia antigen in a sample of urine of a subject, preferably human, and at least one antibody selected from HIV, HCV and *H*. *p*ylori antibody

In one embodiment, the volume of the urine sample is approximately 15 ml.

The object of the present invention is further solved by a method for preparing the urinary fluid immuno-chromatographic detection cup according to the present invention, wherein water-soluble chitosan which is optionally modified is added during the preparation of gold labelled antibody or antigen prior to conjugation of colloid gold with the antibody or antigen.

In one embodiment, the colloidal gold is prepared by reduction of 1% aqueous solution of tetra-chloroauric acid using tri-sodium citrate aqueous solution to produce spheroidal gold particles.

Thus, the present invention provides a urinary multi-parameter rapid immuno-chromatographic detection cup using a unique system for the detection of at least one antigen and/or at least one antibody in a single assay at the same time. This is achieved by using (1) colloidal gold-labelled antibodies and oligo-nucleotide-linked antibodies, and (2) colloidal gold-labelled antigens and oligo-nucleotide-linked antigens. The oligo-nucleotide-linked antibodies and/or antigens are captured by complementary oligo-nucleotides.

One of the advantages of the test device of the present invention is that there is no requirement for the preparation of different rapid immuno-chromatographic test devices to check more than one parameter. Another advantage is that the patient's sample is readily obtainable by non-invasive procedures, namely urine.

High sensitivity of measurements carried out in urine by means of the test device of the present invention is achieved by using a large volume of urine as a sample (about 15 ml urine instead of 17 µl of serum; concentration of IgG in urine is about 1:6000; factor reduction to 1:7) and by using water-soluble chitosan (or modified water-soluble chitosan) as a colour intensity modification agent.

### Detailed Description of the Invention

### Brief Description of the Figures

- Figure 1: shows top and side views of a typical rapid-flow immuno-chromatographic test device in the form of a test strip 101 including a sample pad 102, a conjugate pad 103, a membrane 104, an absorbent pad 105, an adhesive 106, a supporting backing 107, a test zone 108, and a control zone 109.
- Figure 2a: shows an assembly comprising a test strip 201, an absorbent sample pad 202, and an absorbent pad 205.
- Figure 2b: shows an immuno-chromatographic detection cup comprising a sample-collecting container 210, a cap 211, and the assembly shown in Fig. 2a.
- Figure 3a: shows an assembly comprising a test strip 101, an absorbent sample pad 102, a nitrocellulose membrane 104, a conjugate pad 103, an adhesive 106, a supporting backing 107, a control zone 109, and an absorbent pad 105. The conjugate pad 103 contains the gold-labelled antibody 302, oligonucleotide 1 labelled antibody 304, gold labelled antigen 303, oligonucleotide 2 labelled antigen 301. Capturing complementary oligo-nucleotide 1' and 2' are immobilized within sample zone(s) 305 and 306, respectively, onto the nitrocellulose membrane 104.
- Figure 3b: shows the detection test strip of Fib. 3a after the application of a sample 307 that contains a target antibody and a target antigen.

### EXAMPLES

### EXAMPLE 1: Urinary Immuno-chromatographic Detection Cup (Multi-parameter)

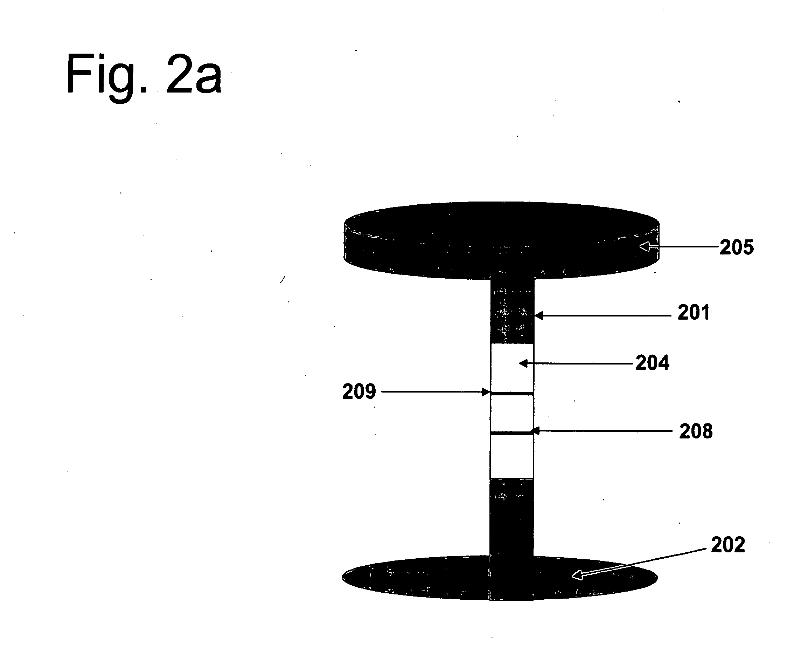

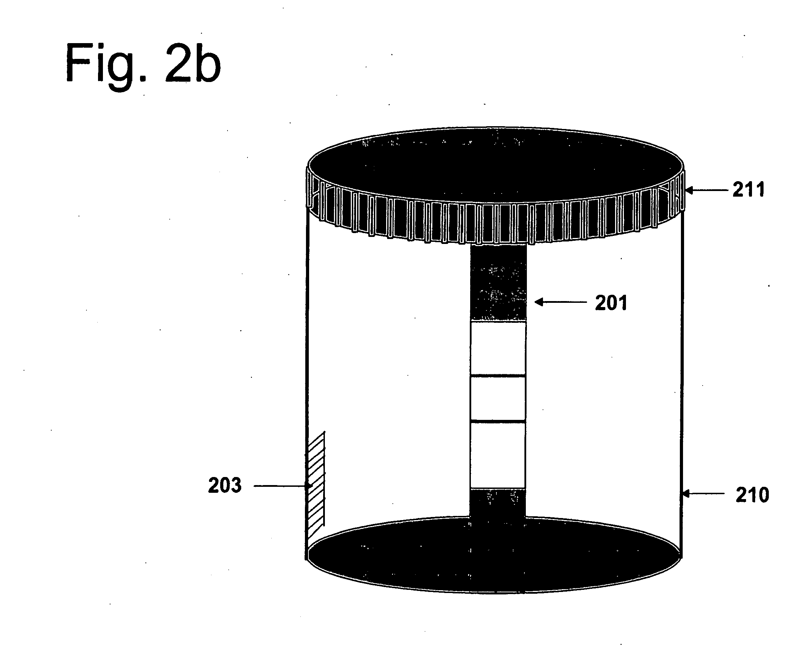

One embodiment of the rapid immuno-chromatographic detection system of the present invention is shown in Fig. 2a, b. The immuno-chromatographic detection cup comprises a sample-collecting container 210, actually the "cup", a cap 211 for closing the container, and a detection test strip 201 inserted into the container 210. The test strip 201 may be placed inside the transparent container wall, and the test result can be read on the outer surface. The test strip 201 comprises a nitrocellulose membrane 204 and is linked via an absorbent pad 202 on the test strip 201 to an absorbent sample pad 202 placed on the bottom of the container 210. The absorbent sample pad 202 is designed to cover the inner surface of the container bottom. At the opposite end, the test strip 201 is linked via an absorbent pad 205 on the test strip to an absorbent pad 205 which is fitted into the cap 211 of the container and is designed to be thick enough to absorb more than 15 ml of sample.

A gold conjugate releasing pad 203 is fixed to the internal surface of the container wall which pad 203 comprises (1a) colloidal gold-labelled antibodies and (1b) oligo-nucleotide-linked antibodies, and (2a) colloidal gold-labelled antigens and (2b) oligo-nucleotide-linked antigens. The gold conjugates will begin releasing from the pad 203 during the urine sample is streaming into the container 210. Release into the sample will increase the possibility of interaction between the conjugates and the analytes, e.g. antibodies and/or antigens.

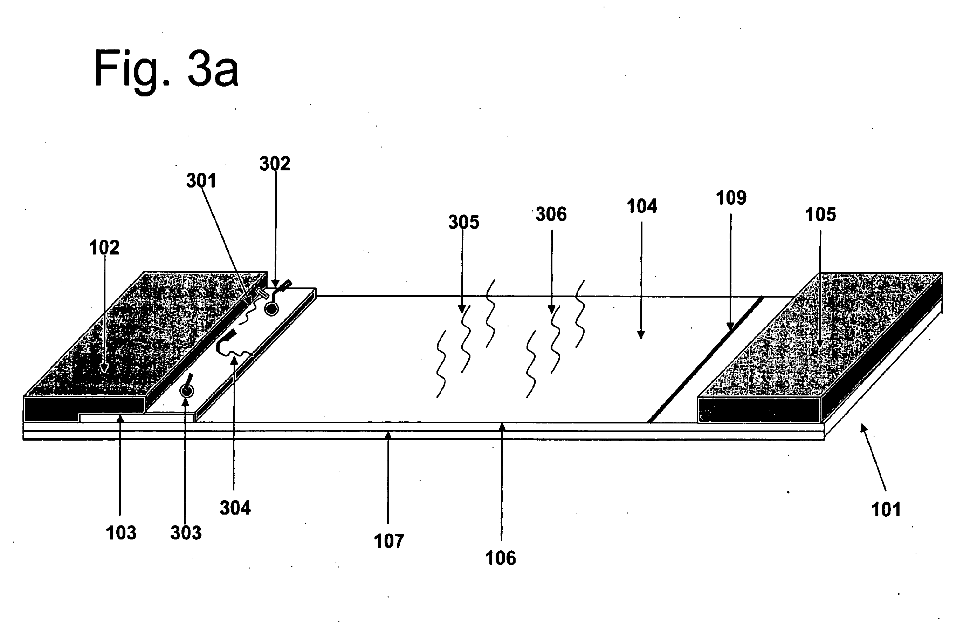

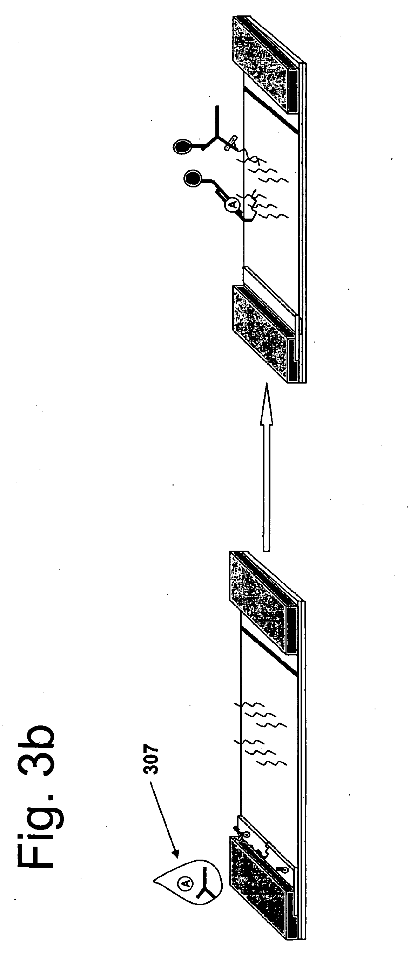

Capturing complementary oligo-nucleotides are immobilized within one or more sample zones 208 onto the nitrocellulose membrane 204 (see also Figure 3a and Figure 3b where it is referred to as 305, 306, i.e. "capturing complementary oligo-nucleotide 1 or 2 sample zones"). If the target antigen is present in the sample, the colloidal gold-labelled antibody and oligo-nucleotide-linked antibody will form a complex with the antigen. If the target antibody is present in the sample, the colloidal gold-labelled antigen and oligo-nucleotide-linked antigen will form a complex with the antibody. Subsequently, the formed complexes migrate to the fixed complementary oligo-nucleotides located at the sample zones 208 and are bound to the solid phase via nucleotide-nucleotide interaction. As a result, at least two types of reactions can be detected on a single test device by the combination of (1) colloidal gold-labelled and oligo-nucleotide-linked antibodies and capturing complementary oligo-nucleotides for detecting an antigen, and (2) colloidal gold-labelled and oligo-nucleotide-linked antigens and capturing complementary oligo-nucleotides for detecting antibodies, wherein antibodies, antigens and complementary oligo-nucleotides are immobilized on a nitrocellulose membrane.

Non-specific antibody and/or antigen is immobilized as a control zone 209 onto the nitrocellulose membrane 204 for non-specific capturing of the gold conjugates (see also Fig. 3). The complementary oligo-nucleotides within the sample zones 208 are for specific capturing of the specific colloidal gold-labelled antibody or antigen-linked oligo-nucleotides.

The antigens used may be synthetic or recombinant. The antibodies used may be polyclonal or monoclonal. In the embodiment shown in Fig. 2a, b the sample zone 208 is realized by a sample line, and the control zone 209 is realized by a control line. Preferably, one sample zone is provided for each parameter. More than one sample zone 208 for each parameter and/or more than one control zone 209 are also contemplated.

Detection of the following diseases is taken into consideration:
(1) Tuberculosis
   Target: LAM (antigen)
   gold-labelled anti-LAM
   oligonucleotide 1-linked anti-LAM
   complementary oligonucleotide 1'
(2) HIV infection
   Target: antibody against envelop protein gp160
   gold-labelled recombinant envelop protein gp 160
   oligonucleotide 2-linked recombinant envelop protein gp160
   complementary oligonucleotide 2'
(3) Malaria
   Target: HRP-II (antigen)
   gold-labelled anti-HRP-II
   oligonucleotide 3-linked anti-HRP-II
   complementary oligonucleotide 3'
(4) HCV infection
   Target: antibody against HCV antigen NS3
   gold-labelled recombinant HCV NS3 antigen
   oligonucleotide 4-linked HCV NS3 antigen
   complementary oligonucleotide 4'
(5) Pneumonia
   Target: pneumolysin PLY (antigen)
   gold-labelled anti-pneumolysin (PLY)
   oligonucleotide 5-linked anti-pneumolysin (PLY) complementary oligonucleotide 5'
(6) *H. pylori* infection
   Target: antibody against *H. pylori* antigen
   gold-labelled *H. pylori* antigen
   oligonucleotide6-linked *H. pylori* antigen
   complementary oligonucleotide 6'

The preparation of oligo-nucleotide-labelled antibody Fab' comprises the following steps⁶:

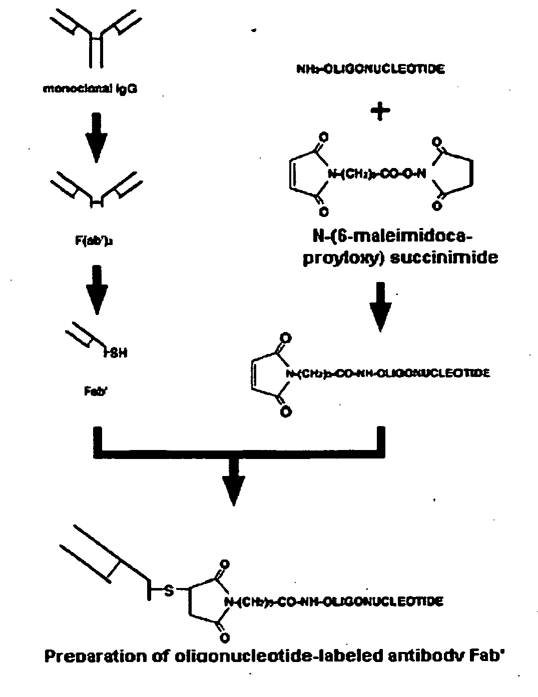

The preparation of oligo-nucleotide-labelled protein comprises the following steps⁷:

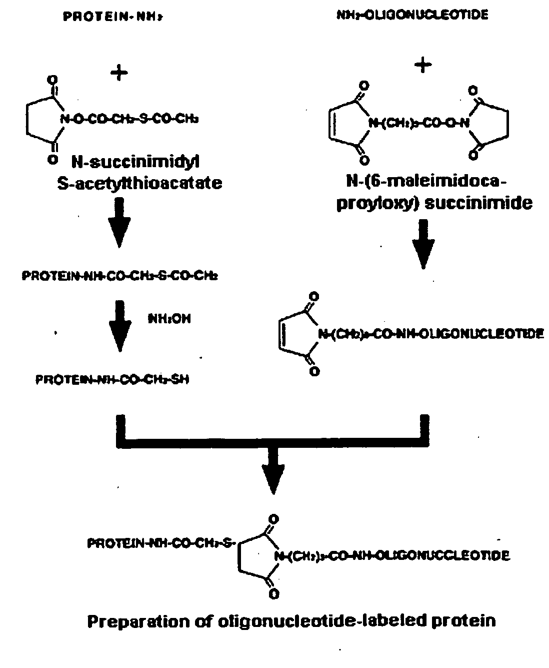

### EXAMPLE 2: Urinary HIV Antibody and Tuberculosis Antigen Detection Cup (Two-Parameters)

The conjugate releasing pad 203 immobilized onto the internal surface of the sample-collecting container 210 comprises gold labelled anti-LAM, oligo-nucleotide 1 linked anti-LAM, gold labelled recombinant envelop protein gp160, oligo-nucleotide 2 linked recombinant envelop protein gp160. A first sample zone 208 (tuberculosis antigen = LAM specific detection zone) comprises complementary oligo-nucleotide 1' immobilized onto the nitrocellulose membrane 204. A second sample zone 208 (HIV antibody specific detection zone) comprises complementary oligo-nucleotide 2' immobilized onto the nitrocellulose membrane. The control zone 209 comprises a mixture of anti-mouse IgG and goat anti-gp160. The first sample zone 208 and control zone 209 turn into purple colour in case that tuberculosis antigen (LAM) is present in the sample, and the second sample zone 208 and control zone 209 turn into purple colour in case that HIV antibody (anti-gp160) is present in the sample; only the control zone turns into purple colour in case of HIV antibody and tuberculosis antigen free sample.

### EXAMPLE 3: Urinary HIV Antibody and Malaria Antigen Detection Cup (Two-Parameters)

The conjugate releasing pad 203 immobilized onto the internal surface of the sample-collecting container 210 comprises gold labelled anti-HRP-II, oligo-nucleotide 3 linked anti-HRP-II, gold labelled recombinant envelop protein gp160, oligo-nucleotide 2 linked recombinant envelop protein gp160. A first sample zone 208 (malaria antigen = HRP-II specific detection zone) comprises complementary oligo-nucleotide 3' immobilized onto the nitrocellulose membrane 204. A second sample zone 208 (HIV antibody specific detection zone) comprises complementary oligo-nucleotide 2' immobilized onto the nitrocellulose membrane. The control zone 209 comprises a mixture of anti-mouse IgG and goat anti-gp160. The first sample zone 208 and control zone 209 turn into purple colour in case that malaria antigen (HRP-II) is present in the sample, and the second sample zone 208 and control zone 209 turn into purple colour in case that HIV antibody (anti-gp160) is present in the sample; only the control zone turns into purple colour in case of HIV antibody and malaria antigen free sample.

### EXAMPLE 4: Urinary HIV Antibody, Malaria Antigen and Tuberculosis Detection Cup (Three-Parameters)

The conjugate releasing pad 203 immobilized onto the internal surface of the sample-collecting container 210 comprises gold labelled anti-LAM, oligo-nucleotide 1 linked anti-LAM, gold labelled anti-HRP-II, oligo-nucleotide 3 linked anti-HRP-II, gold labelled recombinant envelop protein gp160, and oligo-nucleotide 2 linked recombinant envelop protein gp 160. A first sample zone 208 (tuberculosis antigen = LAM specific detection zone) comprises complementary oligo-nucleotide 5' immobilized onto the nitrocellulose membrane 204. A second sample zone 208 (malaria antigen = HRP-II specific detection zone) comprises complementary oligo-nucleotide 3' immobilized onto the nitrocellulose membrane. A third sample zone 208 (HIV antibody specific detection zone) comprises complementary oligo-nucleotide 2' immobilized onto the nitrocellulose membrane. The control zone 209 comprises a mixture of anti-mouse IgG and goat anti-gp160. The first sample zone 208 and control zone 209 turn into purple colour in case that tuberculosis antigen (LAM) is present in the sample, the second sample zone 208 and control zone 209 turn into purple colour in case that malaria antigen (HRP-II) is present in the sample, and the third sample zone 208 and control zone 209 turn into purple colour in case that HIV antibody (anti-gp160) is present in the sample; only the control zone turns into purple colour in case of HIV antibody, tuberculosis antigen and malaria antigen free sample.

### EXAMPLE 5: Urinary HCV Antibody and Malaria Antigen Detection Cup (Two-Parameters)

The conjugate releasing pad 203 immobilized onto the internal surface of the sample-collecting container 210 comprises gold labelled anti-HRP-II, oligo-nucleotide 3 linked anti-HRP-II, gold labelled recombinant HCV NS3 antigen, oligo-nucleotide 4 linked HCV NS3 antigen. A first sample zone 208 (malaria antigen = HPR-II specific detection zone) comprises complementary oligo-nucleotide 3' immobilized onto the nitrocellulose membrane 204. A second sample zone 208 (HCV NS3 antibody specific detection zone) comprises complementary oligo-nucleotide 4' immobilized onto the nitrocellulose membrane. The control zone 209 comprises a mixture of anti-mouse IgG and goat anti-HCV NS3 antigen. The first sample zone 208 and control zone 209 turn into purple colour in case that malaria antigen (HRP-11) is present in the sample, and the second sample zone 208 and control zone 209 turn into purple colour in case that HCV antibody (anti-NS3) is present in the sample; only the control zone turns into purple colour in case of HCV NS3 antibody and malaria antigen free sample.

### EXAMPLE 6: Urinary HCV Antibody and Tuberculosis Antigen Detection Cup (Two-Parameters)

The conjugate releasing pad 203 immobilized onto the internal surface of the sample-collecting container 210 comprises gold labelled anti-LAM, oligo-nucleotide 1 linked anti-LAM, gold labelled recombinant HCV NS3 antigen, oligo-nucleotide 4 linked HCV NS3 antigen. A first sample zone 208 (tuberculosis antigen = LAM specific detection zone) comprises complementary oligo-nucleotide 1' immobilized onto the nitrocellulose membrane 204. A second sample zone 208 (HCV NS3 antibody specific detection zone) comprises complementary oligo-nucleotide 4' immobilized onto the nitrocellulose membrane. The control zone 209 comprises a mixture of anti-mouse IgG and goat anti-HCV NS3 antigen. The first sample zone 208 and control zone 209 turn into purple colour in case that tuberculosis antigen (LAM) is present in the sample, and the second sample zone 208 and control zone 209 turn into purple colour in case that HCV NS3 antibody is present in the sample; only the control zone turns into purple colour in case of HCV NS3 antibody and tuberculosis antigen free sample.

### EXAMPLE 7: Urinary HIV Antibody, HCV Antibody and Malaria Antigen Detection Cup (Three-Parameters)

The conjugate releasing pad 203 immobilized onto the internal surface of the sample-collecting container 210 comprises gold labelled anti-HRP-II, oligo-nucleotide 3 linked anti-HRP-II, gold labelled recombinant HCV NS3 antigen, oligo-nucleotide 4 linked HCV NS3 antigen, gold labelled recombinant envelop protein gp160, and oligo-nucleotide 2 linked recombinant envelop protein gp160. A first sample zone 208 (malaria antigen = HRP-II specific detection zone) comprises complementary oligo-nucleotide 3' immobilized onto the nitrocellulose membrane 204. A second sample zone 208 (HCV NS3 antibody specific detection zone) comprises complementary oligo-nucleotide 4' immobilized onto the nitrocellulose membrane. A third sample zone 208 (HIV gp160 antibody specific detection zone) comprises complementary oligo-nucleotide 2' immobilized onto the nitrocellulose membrane. The control zone 209 comprises a mixture of anti-mouse IgG, goat anti-HCV NS3 antigen and goat anti-gp160. The first sample zone 208 and control zone 209 turn into purple colour in case that malaria antigen (HPR-II) is present in the sample, the second sample zone 208 and control zone 209 turn into purple colour in case that HCV antibody (anti- NS3) is present in the sample, and the third sample zone 208 and control zone 209 turn into purple colour in case that HIV antibody (anti-gp160) is present in the sample; only the control zone turns into purple colour in case of HCV NS3 antibody, HIV gp160 antibody and malaria antigen (HRP-II) free sample.

### EXAMPLE 8: Urinary HIV Antibody, HCV Antibody and Tuberculosis Antigen Detection Cup (Three-Parameters)

The conjugate releasing pad 203 immobilized onto the internal surface of the sample-collecting container 210 comprises gold labelled anti-LAM, oligo-nucleotide 1 linked anti-LAM, gold labelled recombinant HCV NS3 antigen, oligo-nucleotide 4 linked HCV NS3 antigen gold labelled recombinant envelop protein gp160, and oligo-nucleotide 2 linked recombinant envelop protein gp160. A first sample zone 208 (tuberculosis antigen = LAM specific detection zone) comprises complementary oligo-nucleotide 1' immobilized onto the nitrocellulose membrane 204. A second sample zone 208 (HCV NS3 antibody specific detection zone) comprises complementary oligo-nucleotide 4' immobilized onto the nitrocellulose membrane. A third sample zone 208 (HIV gp160 antibody specific detection zone) comprises complementary oligo-nucleotide 2' immobilized onto the nitrocellulose membrane. The control zone 209 comprises a mixture of anti-mouse IgG, goat anti-HCV NS3 antigen and goat anti-gp160. The first sample zone 208 and control zone 209 turn into purple colour in case that tuberculosis antigen (LAM) is present in the sample, the second sample zone 208 and control zone 209 turn into purple colour in case that HCV antibody (anti- NS3) is present in the sample, and the third sample zone 208 and control zone 209 turn into purple colour in case that HIV antibody (anti-gp160) is present in the sample ; only the control zone turns into purple colour in case of HCV antibody (anti- NS3), HIV antibody (anti-gp160) and tuberculosis antigen (LAM) free sample.

### EXAMPLE 9: Urinary HIV Antibody, HCV Antibody, Malaria Antigen and Tuberculosis Antigen Detection Cup (Four-Parameters)

The conjugate releasing pad 203 immobilized onto the internal surface of the sample-collecting container 210 comprises gold labelled anti-LAM, oligo-nucleotide 1 linked anti-LAM, gold labelled anti-HRP-II, oligo-nucleotide 3 linked anti-HRP-II, gold labelled recombinant HCV NS3 antigen, oligo-nucleotide 4 linked HCV NS3 antigen, gold labelled recombinant envelop protein gp160, and oligo-nucleotide 2 linked recombinant envelop protein gp160. A first sample zone 208 (tuberculosis antigen = LAM specific detection zone) comprises complementary oligo-nucleotide 1' immobilized onto the nitrocellulose membrane 204. A second sample zone 208 (malaria antigen = HRP-II specific detection zone) comprises complementary oligo-nucleotide 3' immobilized onto the nitrocellulose membrane 204. A third sample zone 208 (HCV NS3 antibody specific detection zone) comprises complementary oligo-nucleotide 4' immobilized onto the nitrocellulose membrane. A fourth sample zone 208 (HIV gp160 antibody specific detection zone) comprises complementary oligo-nucleotide 2' immobilized onto the nitrocellulose membrane. The control zone 209 comprises a mixture of anti-mouse IgG, goat anti-HCV NS3 antigen and goat anti-gp160. The first sample zone 208 and control zone 209 turn into purple colour in case that tuberculosis antigen (LAM) is present in the sample, the second sample zone 208 and control zone 209 turn into purple colour in case that malaria antigen (HRP-II) is present in the sample, the third sample zone 208 and control zone 209 turn into purple colour in case that HCV antibody (anti-NS3) is present in the sample, and the fourth sample zone 208 and control zone 209 turn into purple colour in case that HIV antibody (anti-gp160) is present in the sample; only the control zone turns into purple colour in case of HCV antibody, HIV antibody, malaria antigen and tuberculosis antigen free sample.

### EXAMPLE 10: Urinary Tuberculosis Antigen, Pneumonia Antigen and H. pylori Antibody Detection Cup (Three-Parameters)

The conjugate releasing pad 203 immobilized onto the internal surface of the sample-collecting container 210 comprises gold labelled anti-LAM, oligo-nucleotide 1 linked anti-LAM, gold labelled anti-pneumolysin (PLY), oligo-nucleotide 5 linked anti-pneumolysin (PLY), and gold labelled *H. pylori* antigen, oligo-nucleotide 6 linked *H. pylori* antigen. A first sample zone 208 (tuberculosis antigen = LAM specific detection zone) comprises complementary oligo-nucleotide 1' immobilized onto the nitrocellulose membrane 204. A second sample zone 208 (pneumonia antigen = PLY specific detection zone) comprises complementary oligo-nucleotide 5' immobilized onto the nitrocellulose membrane. A third sample zone 208 (*H. pylori* antibody specific detection zone) comprises complementary oligonucleotide 6' immobilized onto the nitrocellulose membrane. The control zone 209 comprises a mixture of anti-mouse IgG, and goat anti-*H*. *pylori* antigen. The first sample zone 208 and control zone 209 turn into purple colour in case that tuberculosis antigen (LAM) is present in the sample, the second sample zone 208 and control zone 209 turn into purple colour in case that Pneumonia antigen (PLY) is present in the sample, and the third sample zone 208 and control zone 209 turn into purple colour in case that *H. pylori* antibody is present in the sample; only the control zone turns into purple colour in case of *H. pylori* antibody, tuberculosis antigen and Pneumonia antigen free sample.

### EXAMPLE 11: Amplification of gold conjugate colour intensity

The colloidal gold conjugate colour intensity was amplified using water-soluble chitosan (or modified water-soluble chitosan) as a colour intensity modification agent. The action of water-soluble chitosan (or modified water-soluble chitosan) in connection with the antibodies or antigens is on the colour intensity of the colloidal gold. Chitosan is added during the preparation of colloidal gold, but prior to the conjugation of colloidal gold with the antibodies or the antigens. Water-soluble chitosan (or modified water-soluble chitosan) affect the colour intensity of colloidal gold and so increases the ability of the human eye to identify the colour, and, thus, enables to detect very low concentrations of the analyte. Signal amplification lies in the range of up to 10folds. Colloidal gold could be prepared by the reduction of 1% aqueous solution of tetra-chloroauric acid (HAuCl₄) using tri-sodium citrate aqueous solution to produce spheroidal gold particles. After colloidal gold preparation, water-soluble chitosan (or modified water-soluble chitosan) aqueous solution was added with a suitable volume and concentration to convert colour from purple to violet pending on the volume and concentration of the added modification solution.

### References

(1) World Health Organization, HIV assays: operational characteristics (Phase I). Report 13: urine specimens, oral fluid (saliva) specimens. [Material originally distributed as WHO/BCT/02.08]
(2) Journal of Microbiological Methods 54: 411-52, 2001
(3) J Chandler, N Robinson, and K Whiting, "Handling False Signals in Gold-Based Rapid Tests", IVD Technology 7, no. 2 (2001): 34-45; http://www.devicelink.com/ivdt/archive/01/03/002.html.
(4) TC Tisone et al., "Image Analysis for Rapid-Flow Diagnostics", IVD Technology 5, no. 5 (1999): 52-58; http://www.devicelink.com/ivdt/archive/99/09/010.html.
(5) J Chandler, T Gurmin, and N Robinson, "The Place of Gold in Rapid Tests", IVD Technology 6, no. 2 (2000): 37-49; http://www.devicelink.com/ivdt/archive/00/03/004.html
(6) Oku, Y., Uesaka, Y., Hirayama, T., Takeda, Y., 1988. Development of a highly sensitive Bead-ELISA to detect bacterial protein toxins. Microbiol. Immunol. 32, 807.
(7) Duncan, R.J.S., Weston, P.D., Wrigglesworth, R., 1983. A new reagent which may be used to introduce sulfhydryl groups into proteins, and its use in the preparation of conjugates for immunoassay. Anal. Biochem. 132, 68.
(8) A Sugunan, C Thanachayanont, J Dutta, JG Hilborn. Heavy-metal ion sensors using chitosan-capped gold nanoparticles, Science and Technology of Advanced Materials 6 (2005) 335-340.

## Claims

1. A urinary immuno-chromatographic detection cup comprising:
(a) a sample-collecting container;
(b) at least one conjugate releasing pad, which conjugate releasing pad comprises at least one gold labelled antibody X and at least one oligo-nucleotide linked antibody X', wherein both antibodies are directed against the same antigen, and at least one gold labelled antigen Y and at least one oligo-nucleotide linked antigen Y', wherein both antigens are recognized by the same antibody;
(c) at least one test means inside the container separated from the conjugate releasing pad, which test means comprises at least one region comprising an oligo-nucleotide complementary to the oligo-nucleotide linked to the antibody X', at least one region comprising an oligo-nucleotide complementary to the oligo-nucleotide linked antigen Y', and at least one region comprising a control antibody and/or a control antigen;
(d) at least two sample absorbent pads linked to the test device at different positions, wherein a first sample absorbent pad is located at the bottom, and a second sample absorbent pad is located at the top of the container, and wherein the sample absorbent pad at the top has a capacity of absorbing at least 15 ml of sample.

2. The detection cup according to claim 1, wherein the antibodies X and X' are directed against an antigen selected from tuberculosis antigen, malaria antigen and pneumonia antigen.

3. The detection cup according to claim 2, wherein the tuberculosis antigen is LAM, the malaria antigen is HRP-II, and the pneumonia antigen is PLY.

4. The detection cup according to any of the preceding claims, wherein the control antibody is anti-mouse IgG.

5. The detection cup according to claim 1, wherein the antigens Y and Y' are selected from envelop protein gp 160 from HIV, NS3 antigen from HCV, and *H. pylori* antigen.

6. The detection cup according to any of the preceding claims, wherein the test device further comprises a nitrocellulose membrane on which the complementary oligonucleotides are immobilized.

7. The detection cup according to any of the preceding claims, wherein the conjugate releasing pad is located at the internal surface of the container wall.

8. The detection cup according to any of the preceding claims, wherein the sample absorbent pad at the top is fitted into a container cap.

9. The detection cup according to any of the preceding claims, wherein the conjugate releasing pad further comprises water-soluble chitosan which is optionally modified.

10. A use of the urinary immuno-chromatographic detection cup according to any of claims 1 to 9 for detecting at least one antigen selected from tuberculosis antigen, malaria antigen and pneumonia antigen in a sample of urine of a subject, preferably human, and at least one antibody selected from HIV antibody, HCV antibody and *H. py*lori antibody.

11. The use according to claim 10, wherein the volume of the urine sample is 15 ml.

12. A method for preparing the urinary fluid immuno-chromatographic detection cup according to any of claims 1 to 9, wherein water-soluble chitosan which is optionally modified is added during the preparation of gold labelled antibody X or gold labelled antigen Y prior to conjugation of colloid gold with the antibody or antigen.

13. The method according to claim 12, wherein the colloidal gold is prepared by reduction of 1% aqueous solution of tetra-chloroauric acid using tri-sodium citrate aqueous solution to produce spheroidal gold particles.

## Patentansprüche

1. Becher zum immunchromatographischen Nachweis im Urin, der Folgendes umfasst:
(a) einen Probensammelbehälter;
(b) mindestens ein Konjugat freisetzendes Pad, wobei das Konjugat freisetzende Pad mindestens einen Gold-markierten Antikörper X und mindestens einen Oligonukleotid-verbundenen Antikörper X' umfasst, wobei beide Antikörper gegen dasselbe Antigen gerichtet sind, und mindestens ein Goldmarkiertes Antigen Y und mindestens ein Oligonukleotid-verbundenes Antigen Y', wobei beide Antigene durch denselben Antikörper erkannt werden;
(c) mindestens eine Testvorrichtung in dem Behälter, die von dem Konjugat freisetzenden Pad getrennt ist, wobei die Testvorrichtung mindestens eine Region umfasst, die ein Oligonukleotid umfasst, das zu dem Oligonukleotid, das mit dem Antikörper X' verbunden ist, komplementär ist, mindestens eine Region, die ein Oligonukleotid umfasst, das zu dem Oligonukleotid, das mit dem Antigen Y' verbunden ist, komplementär ist, und mindestens eine Region, die einen Kontrollantikörper und/oder ein Kontrollantigen umfasst;
(d) mindestens zwei Probe absorbierende Pads, die mit der Testvorrichtung an verschiedenen Positionen verbunden sind, wobei ein erstes Probe absorbierendes Pad am Boden angeordnet ist und ein zweites Probe absorbierendes Pad oben am Behälter angeordnet ist, und wobei das oben angeordnete Probe absorbierende Pad eine Aufnahmefähigkeit zum Absorbieren von mindestens 15 ml Probe aufweist.

2. Nachweisbecher gemäß Anspruch 1, wobei die Antikörper X und X' gegen ein Antigen gerichtet sind, das ausgewählt ist aus Tuberkuloseantigen, Malariaantigen und Pneumonieantigen.

3. Nachweisbecher nach Anspruch 2, wobei das Tuberkuloseantigen LAM, das Malariaantigen HRP-II und das Pneumonieantigen PLY ist.

4. Nachweisbecher nach einem der vorangehenden Ansprüche, wobei der Kontrollantikörper anti-Maus-IgG ist.

5. Nachweisbecher nach Anspruch 1, wobei die Antigene Y und Y' ausgewählt sind aus Hüllprotein gp160 von HIV, NS3-Antigen von HCV und *H. pylori*-Antigen.

6. Nachweisbecher nach einem der vorangehenden Ansprüche, wobei die Testvorrichtung weiterhin eine Nitrozellulosemembran umfasst, an der die komplementären Oligonukleotide immobilisiert sind.

7. Nachweisbecher nach einem der vorangehenden Ansprüche, wobei das Konjugat freisetzende Pad an der inneren Oberfläche der Behälterwand angeordnet ist.

8. Nachweisbecher nach einem der vorangehenden Ansprüche, wobei das oben angeordnete Probe absorbierende Pad in eine Behälterkappe eingepasst ist.

9. Nachweisbecher nach einem der vorangehenden Ansprüche, wobei das Konjugat freisetzende Pad weiterhin wasserlösliches Chitosan umfasst, das wahlweise modifiziert ist.

10. Verwendung des Bechers zum immunchromatographischen Nachweis im Urin nach einem der Ansprüche 1 bis 9 zum Nachweisen von mindestens einem Antigen, ausgewählt aus Tuberkuloseantigen, Malariaantigen und Pneumonieantigen, in einer Urinprobe eines Subjekts, vorzugsweise eines Menschens, und mindestens einem Antikörper, ausgewählt aus HIV-Antikörper, HCV-Antikörper und *H. pylori*-Antikörper.

11. Verwendung nach Anspruch 10, wobei das Volumen der Urinprobe 15 ml beträgt.

12. Verfahren zum Herstellen des Bechers zum immunchromatographischen Nachweis in Urinflüssigkeit nach einem der Ansprüche 1 bis 9, wobei wasserlösliches Chitosan, das wahlweise modifiziert ist, während der Herstellung von Gold-markiertem Antikörper X oder Gold-markiertem Antigen Y vor Konjugation von kolloidalem Gold mit dem Antikörper oder Antigen zugegeben wird.

13. Verfahren nach Anspruch 12, wobei das kolloidale Gold durch Reduktion einer 1 %igen wässrigen Lösung von Tetrachlorogoldsäure unter Verwendung von wässriger Trinatriumcitratlösung hergestellt wird, um spheroidale Goldpartikel herzustellen.

## Revendications

1. Récipient pour la détection immuno-chromatographique urinaire, comprenant :
(a) un récipient de recueil d'échantillon ;
(b) au moins un tampon de libération de conjugué, ledit tampon de libération de conjugué comprenant au moins un anticorps X marqué à l'or et au moins un anticorps X' lié à un oligonucléotide, les deux anticorps étant dirigés contre le même antigène, et au moins un antigène Y marqué à l'or et au moins un antigène Y' lié à un oligonucléotide, les deux antigènes étant reconnus par le même anticorps ;
(c) au moins un moyen de test à l'intérieur du récipient, séparé du tampon de libération du conjugué, ledit moyen de test comprenant au moins une région comprenant un oligonucléotide complémentaire à l'oligonucléotide lié à l'anticorps X', au moins une région comprenant un oligonucléotide complémentaire à l'antigène Y' lié à l'oligonucléotide, et au moins une région comprenant un anticorps témoin et/ou un antigène témoin ;
(d) au moins deux tampons absorbant de l'échantillon liés au dispositif de test à différentes positions, un premier tampon absorbant de l'échantillon étant situé au fond et un deuxième tampon absorbant de l'échantillon étant situé en haut du récipient et le tampon absorbant de l'échantillon supérieur ayant une capacité d'absorption d'au moins 15 ml d'échantillon.

2. Récipient de détection selon la revendication 1, dans lequel les anticorps X et X' sont dirigés contre un antigène choisi parmi l'antigène de la tuberculose, l'antigène du paludisme et l'antigène de la pneumonie.

3. Récipient de détection selon la revendication 2, dans lequel l'antigène de la tuberculose est LAM, l'antigène du paludisme est HRP-II et l'antigène de la pneumonie est PLY.

4. Récipient de détection selon l'une quelconque des revendications précédentes, dans lequel l'anticorps témoin est une IgG anti-souris.

5. Récipient de détection selon la revendication 1, dans lequel les antigènes Y et Y' sont choisis parmi la protéine d'enveloppe gp 160 de VIH, l'antigène NS3 de HCV et l'antigène de *H. pylori*.

6. Récipient de détection selon l'une quelconque des revendications précédentes, dans lequel le dispositif de test comprend une membrane de nitrocellulose sur laquelle les oligonucléotides complémentaires sont immobilisés.

7. Récipient de détection selon l'une quelconque des revendications précédentes, dans lequel le tampon de libération de conjugué est situé sur la surface interne de la paroi du conteneur.

8. Récipient de détection selon l'une quelconque des revendications précédentes, dans lequel le tampon absorbant l'échantillon situé au niveau supérieur est fixé dans un couvercle du récipient.

9. Récipient de détection selon l'une quelconque des revendications précédentes, dans lequel le tampon de libération de conjugué comprend en outre du chitosan hydrosoluble qui est éventuellement modifié.

10. Utilisation du récipient de détection immuno-chromatographique urinaire selon l'une quelconque des revendications 1 à 9, pour détecter au moins un antigène choisi parmi l'antigène de la tuberculose, l'antigène du paludisme et l'antigène de la pneumonie dans un échantillon d'urine d'un sujet, de préférence, un être humain, et au moins un anticorps choisi parmi un anticorps de VIH, un anticorps de HCV et un anticorps de *H. pylori.*

11. Utilisation selon la revendication 10, dans laquelle le volume de l'échantillon d'urine est de 15 ml.

12. Procédé de préparation d'un récipient de détection immuno-chromatographique dans le fluide urinaire selon l'une quelconque des revendications 1 à 9, dans lequel le chitosan hydrosoluble qui est éventuellement modifié est ajouté pendant la préparation de l'anticorps X marqué à l'or ou de l'antigène Y marqué à l'or avant la conjugaison de l'or colloïdal avec l'anticorps ou l'antigène.

13. Procédé selon la revendication 12, dans lequel l'or colloïdal est préparé par réduction d'une solution aqueuse à 1 % d'acide tétra-chloro-aurique en utilisant une solution aqueuse de citrate trisodique pour produire des particules d'or sphéroïdales.
